(19) Europäisches Patentamt
European Patent Office
Office européen des brevets

(11) Veröffentlichungsnummer: **0 126 934**
**B1**

(12) # EUROPÄISCHE PATENTSCHRIFT

(45) Veröffentlichungstag der Patentschrift: **05.11.86**

(51) Int. Cl.⁴: **C 07 C 157/02**, C 07 D 295/20

(21) Anmeldenummer: **84104207.0**

(22) Anmeldetag: **13.04.84**

(54) **Verfahren zur Herstellung von 1,1-disubstituierten Thioharnstoffen.**

(30) Priorität: **21.04.83 DE 3314435**

(43) Veröffentlichungstag der Anmeldung: **05.12.84 Patentblatt 84/49**

(45) Bekanntmachung des Hinweises auf die Patenterteilung: **05.11.86 Patentblatt 86/45**

(84) Benannte Vertragsstaaten: **BE CH DE FR GB LI NL**

(56) Entgegenhaltungen: **DD-A-100 467**

(73) Patentinhaber: **BASF Aktiengesellschaft, Carl-Bosch- Strasse 38, D-6700 Ludwigshafen (DE)**

(72) Erfinder: **Seybold, Guenther, Dr., Friedrich- Ebert-Strasse 14, D-6708 Neuhofen (DE)**
Erfinder: **Dimmler, Manfred, Dr., Nordring 53, D-6701 Dannstadt- Schauernheim (DE)**
Erfinder: **Stange, Andreas, Dr., Kantstrasse 8, D-6800 Mannheim 1 (DE)**

**EP 0 126 934 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Herstellung von 1,1-disubstituierten Thioharnstoffen durch Umsetzung von Säurehalogeniden mit Isothiocyanaten in Gegenwart von organischen Lösungsmitteln bei höchstens 15°C, dann Umsetzung der so gebildeten N-Acylisothiocyanate mit sekundären Aminen bei einer Temperatur von höchstens 15°C während höchstens 90 Minuten und dann während mindestens 180 Minuten bei einer Temperatur von 20 bis 35°C, Abtrennung der organischen Lösungsmittel, Zugabe von 5 bis 38 gew.%igen wäßrigen Säuren in einem Verhältnis von höchstens 4 Äquivalenten Säure je Mol Stoff VI zu den so gebildeten 1,1-disubstituierten 3-Acylthioharnstoffen und Umsetzung des Gemischs bei einer Temperatur von 20 bis 100°C.

Es ist aus der DD-PS 100 487 bekamnt, daß man durch Umsetzung von Säurehalogeniden mit Isothiocyanaten in Gegenwart von organischen Lösungsmitteln, Umsetzung der gebildeten N-Acylisothiocyanate mit sekundären Aminen und Umsetzung der gebildeten 1,1-disubstituierten 3-Acylthioharnstoffe mit Säure, 1,1-disubstituierte Thioharnstoffe herstellen kann. Es wird beschrieben, daß nach der Umsetzung des Acylisothiocyanats mit dem Amin das Reaktionsprodukt ohne Entfernung des organischen Lösungsmittels zunächst mit verdünnter wäßriger Mineralsäure behandelt und dann durch Kochen mit vorzugsweise konzentrierter Salzsäure gespalten wird. Als Lösungsmittel werden polare, organische, hydroxylgruppenfreie Lösungsmittel, z.B. auch Essigester oder Dimethylfotmamid, genannt, aber in den Beispielen nur Aceton und Acetonitril verwendet. Wie Beispiel 1 zeigt, wird die Umsetzung des Säurehalogenids mit dem Isothiocyanat während 2 Stunden durch Kochen am Rückfluß durchgeführt. In allen Beispielen wird das Reaktionsgemisch der Herstellung der 3-Acylthioharmstoffe in verdünnte bzw. konzentrierte Salzsäure eingegossen und das Gemisch dann eimgedampft bzw. erhitzt. Im Beispiel 5 wird das Gemisch nach Eintragen in verdünnter Salzsäure über Nacht stehen gelassen und erst dann mit konzentrierter Salzsäure gekocht. Nach diesem Verfahren werden nur, wie die Beispiele zeigen, Ausbeuten von 7,7 bis 40,5 % erzielt. Nachteilig ist, daß bei Addition des sekundären Amins an das N-Acylisothiocyanat sowohl eine Reaktion an der N=C=S-Gruppierung als auch an der C=O-Gruppe erfolgt. Beispiel 4 zeigt eine Ausbeute von 48 % an Benzoylmorpholinothioharnstoff, der durch die Spaltung nur zu 55 % in den Endstoff umgewandelt wird. Ebenso ist nachteilig, daß das als Lösungsmittel verwendete Aceton nicht wiedergewonnen, sondern mit dem Reaktionsgemisch in die Salzsäure eingeleitet wird; es entstehen Köndensationsprodukte des Acetons, die den Endstoff verunreinigen.

Es wurde nun gefunden, daß man 1,1-disubstituierte Thioharnstoffe der Formel

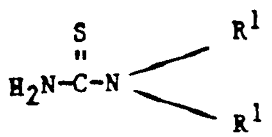

I,

worin die einzelnen Reste $R^1$ gleich oder verschieden sein können und jeweils einem aliphatischen, cycloaliphatischen, araliphatischen oder aromatischem Rest oder zusammen mit den benachbarten Stickstoffatom Glieder eines heterocyclischen Ringes, der noch ein heiteres Stickstoffatom oder ein Sauerstoffatom entbalten kann, bedeuten, durch Umsetzung von Säurebaloseniden mit Isothiocyanaten in Gegenwart von organischen Lösungsmitteln, Umsetzung der gebildeten N-Acylisothiocyanate mit sekundären Aminen und Umsetzung der gebildeten 1,1-disubstituierten 3-Acylthioharnstoffe mit Säure und Isolierung des Endstoffs aus dem Gemisch in bekannter Weise, vorteilhaft erhält, wenn man

a) in einer 1. Stufe Säurehalogenide der Formel

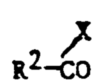

II,

worin $R^2$ einen verzweigten aliphatischen Rest, einen cycloaliphatischen Rest, einen am Benzolringen substituierten aromatischen Rest, einem araliphatischen Rest oder einen aromatisch-cycloaliphatischen Rest bedeutet, und X ein Halogenatom bezeichnet, mit Isothiocyanaten der Formel

$$R^3-S-C\equiv N \qquad III,$$

worin $R^3$ für ein Alkaliatom oder eine Ammoniumgruppe steht, in Gegen-Hart von organischen Lösungsmittel bei einer Temperatur von höchstens 15°C umsetzt, dann

b) in einer 2. Stufe die so gebildeten N-Acylisothiocyanate der Formel

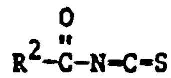

IV,

worin $R^2$ die vorgenannte Bedeutung besitzt, mit sekundären Aminen der Formel

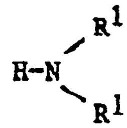

V,

worin $R^1$ die vorgenannte Bedeutung besitzt, bei einer Temperatur von höchstens 15° C während höchstens 90 Minuten umsetzt, dann

c) in einer 3. Stufe das Reaktionsgemisch während mindestens 180 Minuten bei einer Temperatur von 20 bis 35° C hält, dann

d) in einer 4. Stufe organische Lösungsmittel aus dem Reaktionsgemisch durch Destillation abtrennt, dann

e) in einer 5. Stufe zu den so gebildeten 1,1-disubstituierten 3-Acyl-thioharnstoffen der Formel

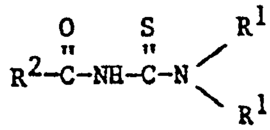

VI,

worin $R^1$ und $R^2$ die vorgenannte Bedeutung besitzen, 5 bis 38 gew.%ige wäßrige Säuren in einem Verhältnis von höchstens 4 Äquivalenten Säure je Mol Stoff VI zugibt und das Gemisch bei einer Temperatur von 20 bis 100°C

umsetzt.

Die Umsetzung kann für den Fall der Verwendung von Natriumisothiocyanat und Isobuttersäurechlorid, Diethylamin und als Säure Salzsäure durch die folgenden Formeln beschrieben werden:

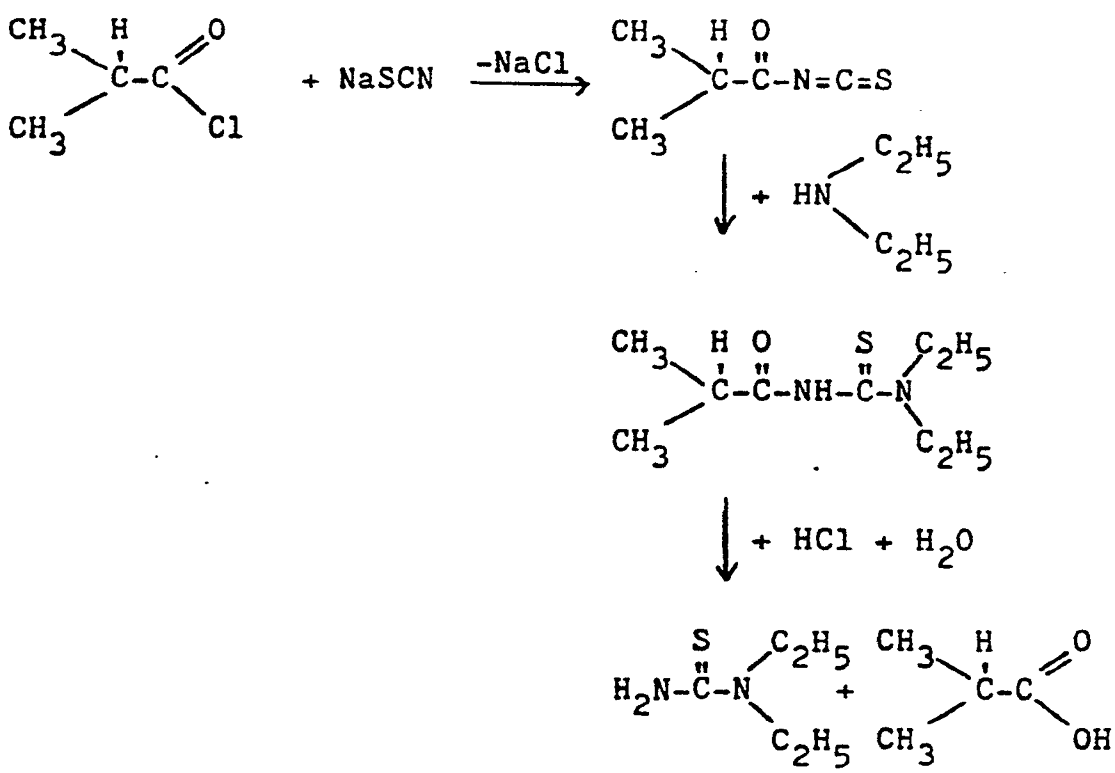

Im Vergleich zu dem bekannten Verfahren liefert das Verfahren nach der Erfindung auf einfacherem und wirtschaftlicherem Wege 1,1-disubstituierte Thioharnstoffe in besserer Ausbeute und Reinheit. Alle diese vorteilhaften Ergebnisse sind im Hinblick auf den Stand der Technik überraschend. Es war mit Bezug auf das bekannte Verfahren nicht zu erwarten, daß die tiefen Reaktionstemperaturen (a und b), die Verweilzeit (b), die zweistufige Umsetzung in b) und c) mit Amin und insbesondere die Abtrennung des Lösungsmittels vom Reaktionsgemisch (d) vor der Spaltung und die Zugabe der Säure zum Reaktionsgemisch (e) bei dem erfindungsgemäßen Verfahren die genannten technischen Vorteile bieten.

Die Ausgangsstoffe II und III bzw. IV und V können miteinander im Überschuß jeder Komponente zur anderen oder vorteilhaft in stöchiometrischer Menge, zweckmäßig in einem Verhältnis von 0,99 bis 1 01 Mol Ausgangsstoff III und/oder von 1 bis 1,1 Mol Stoff V je Mol Ausgangsstoff II umgesetzt werden. Bevorzugte Ausgangsstoffe II, III, Stoffe IV, V und dementsprechend bevorzugte Endstoffe I sind solche, in deren Formeln die einzelnen Reste $R^1$ gleich oder verschieden sein können und jeweils einen unverzweigten oder verzweigten Alkylrest mit 1 bis 10, insbesondere 1 bis 4 Kohlenstoffatomen, einen Cycloalkylrest mit 5 bis 8 Kohlenstoffatomen, einen Aralkylrest oder Alkylarylrest mit 7 bis 12 Kohlenstoffatomen, einen Phenylrest oder zusammen mit dem benachbarten Stickstoffatom Glieder eines 5- oder 8-gliedrigen, heterocyclischen Ringes, der noch ein weiteres Stickstoffatom oder ein Sauerstoffatom enthalten kann, bedeuten, $R^2$ einen verzweigten Alkylrest mit 4 bis 15, vorzugsweise 4 bis 10 Kohlenstoffatomen einen gegebenenfalls durch 1 bis 4 Alkylgruppen mit jeweils 1 bis 4 Kohlenstoffatomen und/oder 1 bis 2 Phenylgruppen substituierten, monocyclischen oder polycyclischen, bevorzugt bicyclischen oder tricyclischen Cycloalkylrest mit 5 bis 18, vorteilhaft 8 bis 10 Kohlenstoffatomen, einen an mehreren Benzolringen, vorteilhaft einem Benzolring durch 1 bis 3 Alkylgruppen mit jeweils 1 bis 4 Kohlenstoffatomen substituierten Phenylrest oder substituierten Naphthylrest, einen Aralkylrest mit 7 bis 12 Kohlenstoffatomen bezeichnet, X für ein Bromatom und insbesondere ein Chloratom steht und $R^3$ ein Natrium- oder Kaliumatom bedeutet. Die vorgenannten Reste können noch durch unter Reaktionsbedingungen inerte Gruppen, z.B. Alkylgruppen mit 1 bis 4 Kohlenstoffatomen, substituiert sein.

So kommen z.B. folgende Ausgangsstoffe II in Betracht: die Säurechloride der Isobuttersäure, Isocapronsäure, Isocaprylsäure, Hesperitinsäure, Pivalinsäure, Isovaleriansäure, 3,5,5-Trimethyl-n-hexansäure, 2-Ethylbuttersäure, 2-Ethylvaleriansäure, 2-Ethylcapronsäure, 2-Ethylcaprylsäure, 2-Ethylheptylsäure, 2-Ethylnonylsäure, 2-Ethyldecylsäure, 2-Methylbuttersäure, 2-Methylvaleriansäure, 2-Methylcapronsäure, 2-Methylcaprylsäure, Z-Methylheptylsäure, 2-Methylnonylsäure, 2-Methyldecylsäure, 2,6-, 2,4-, 3,5-, 2,5-Dimethylbenzoesäure, 4-Isopropylbenzoesäure, Phthalsäuremönomethylester, Isophthalsäuremonomethylester, Terephthalsäuremönomethylester, 2,4,6-Trimethylbenzoesäure, Phenylessigsäure, Phenylpropionsäure, Adamantan-1-carbonsäure, Tricyclodecan-2-carbonsäure, Tricyclodecan-3-carbonsäure, Tricyclodecan-5-carbonsäure, Cyclohexan-carbonsäure, Cyclopentancarbonsäure, 1-Phenylcyclohexancarbonsäure, 1-Methylcyclohexancarbonsäure, 1-Ethylcyclohexancarbonsäure, 1-Phenylcyclopentancarbonsäure, enrsprechende Säurebromide.

Bevorzugte Ausgangsstoffe III sind Kaliumisothiocyanat und insbesondere Natriumisothiocyanat.

Als Ausgangsstoffe V sind z.B. folgende sekundären Amine geeignet: Dimethyl-, Diethyl-, Dipropyl-, Diisopropyl-, Dibutyl-, Di-sek.-butyl-, Di-tert.-butyl-, Diisobutyl-, Dicyclohexyl-, Dicyclopentyl-, Dibenzyl-, Diphenyl-amin, Methylethylamin, Pyrrolidin, Piperidin, Morpholin, Imidazolidin, Pyrazolidin.

Die Umsetzung wird in Stufe a) bei einer Temperatur von höchstens 15, zweckmäßig -15 bis +15, bevorzugt -10 bis +10°C, in Stufe b) von höchstens 15, zweckmäßig -15 bis +15, bevorzugt -10 bis +15°C, in Stufe c) von 20 bis 35°C, bevorzugt 20 bis 30°C, in Stufe ensprechend der Destillationstemepratur des Lösungsmittels drucklos unter Unterdruck oder unter Überdruck, kontinuierlich oder diskontinuierlich durchgeführt. Die Reaktionszeiten betragen in Stufe b) höchstens 90, zweckmäßig 1 bis 90, bevorzugt 50 bis 90 Minuten, zweckmäßig in Stufe a) 60 bis 600, bevorzugt 120 bis 360 Minuten, zwecknäßig in Stufe c) 180 bis 720, bevorzugt 240 bis 540 Minuten.

Man kann zwar in jeder Stufe a), b) und/oder c) frisches Lösungsmittel zufügen bzw. nach der Reaktion abtrennen, jedoch wird nan schon aus Zweckmäßigkeitsgründen das gesamte Lösungsmittel in der Stufe a) verwenden und einbadig dann in b) und c) ohne Abtrennung oder Zugabe von Lösungsmitteln umsetzen. Gegebenenfalls kann das Amin V in Form seiner Lösung, vorzugsweise 20 bis 90 gew.%iger Lösung in einem organischen Lösungsmittel, zweckmäßig die nachgenannten, in denen die Stufen a), b) und c) jeweils durchgeführt werden, zugesetzt werden.

Man verwendet unter den Reaktionsbedingungen inerte, organische Lösungsmittel. Als Lösungsmittel kommen z.B. in Frage: aromatische Kohlenwasserstoffe, z.B. Toluol, Ethylbenzol, o-, m-, p-Xylol, Isopropylbenzol, Ether, z.B. Ethylpropylether, Methyl-tert.-butylether, n-Butylethylether, Di-n-butylether, Diisobutylether, Diisopropylether, Diethylether, Dioxan; Ketone wie Methylethylketon, Aceton, Diisopropylketon, Diethylketon, Methylisobutylketon, Cyclohexanon, Diisobutylketon; Ester wie Methylacetat, Butylacetat, Ethylformiat, Essigester, Nitrile wie Acetonitril; aliphatische oder cycloaliphatische Kohlenwasserstoffe, z.B. Pentan, Heptan, Benzinfraktionen innerhalb eines Siedepunktintervalls von 70 bis 190°C, Cyclohexan, Methylcyclohexan, Dekalin, Petrolether, Hexan, Ligroin, und entsprechende Gemische. Zweckmäßig verwendet man das Lösungsmittel in einer Menge von 300 bis 10 000 G w.%, vorzugsweise von 300 bis 1000 Gew.%, bezogen auf Ausgangsstoff II.

In Stufe d) wird Lösungsmittel, zweckmäßig 50 bis 100, vorteilhaft 90 bis 100, vorzugsweise 98 bis 100, insbesondere 100 Gew.% der gesamten Lösungsmittelmenge, die in den Stufen b), c) und in der Regel a)

zugegeben werden, abgetrennt. Unter Lösumgsmittel wird hier das zusätzliche, organische Lösungsmittel und nicht ein auch als Lösungsmittel verwendeter, unumgesetzter Ausgangsstoff II oder Amin V verstanden. Die Destillation kann in üblicher Weise, z.B. in einer Destillationskolonne, Rektifikationskolonne oder durch Wasserdampfdestillation, erfolgen.

Nach der Abtrennung des Lösungsmittels in Stufe d) verbleibt ein Rückstand, der im wesentlichen dem Stoff VI entspricht. Die Behandlung des Reaktionsgemisches nach der Destillation wird in Gegenwart von höchstens 4 Äquivalentem Säure, vorteilhaft mit einer Menge von 2,3 bis 3,5, insbesondere von 2,3 bis 3 Äquivalenten Säure, bezogen auf 1 Mol Ausgangsstoff VI, durchgeführt. Es können insbesondere anorganische Säuren verwendet werden. Anstelle einbasischer Säuren können auch äquivalente Mengen mehrbasischer Säuren zur Anwendung gelangen. Beispielsweise sind folgende Säuren geeignet: Chlorwasserstoff, Bromwasserstoff, Schwefelsäure, Phosphorsäure, saure Ionenaustauscher; oder entsprechende Gemische. Die Säuren werden im allgemeinen in Gestalt ihrer wäßrigen, 5 bis 38, zwecknäßig 5 bis 36 gew.%igen Lösung angewendet, z.B. 32 bis 36 gew.%igen Salzsäure, 5 bis 16 gew.%igen Schwefelsäure oder 5 bis 25 gew.%igen Fhosphorsäure. Bevorzugt sind Schwefelsäure, Phosphorsäure und insbesondere Salzsäure. Der pH-Wert der U setzung beträgt zwischen 1 und 3, vorzugsweise von 1 bis 2, insbesondere von 1 his 1,5.

Die Umsetzung in Stufe e) wird im allgemeinen bei einer Temperatur von bis 100, zweckmäßig 60 bis 100°C, vorteilhaft 60 bis 95°C, vorzugsweise von 80 bis 95°C, unter Unterdruck oder unter Überdruck, vorteilhaft drucklos, kontinuierlich oder diskontinuierlich durchgeführt. Die Reaktionszeit beträgt zweckmäßig in Stufe e) 45 bis 300, insbesondere 60 bis 120 Minuten. Es ist ein erfindungsgemäßes Merkmal, daß man die Säure dem Reaktionsprodukt bzw. Rückstand der Stufe d) und nicht umgekehrt zugibt. Man kann die Spaltung während der ganzen Stufe e) bei einer einzigen, tiefen, z.B. 40 bis 60°C, oder vorzugsweise höheren Temperatur, z.B. 85 bis 100°C oder zweckmäßiger während der Stufe e) unter Erhitzen von einer tieferen auf eine höhere Temperatur, z.B. von 60 auf 95°C, durchführen. In einer bevorzugten Ausführungsform der Stufe e) erhitzt man während der Reaktion während 10 bis 30 Minuten von 20 auf 90 bis 95°C und hält gegebenenfalls die Temperatur während 60 bis 120 Minuten bei 90 bis 95°C.

Die Reaktion kann wie folgt durchgeführt werden: a) Ein Gemisch von Ausgangsstoff III, Säurehalogenid II und organischem Lösungsmittel wird während der Reaktionszeit bei der Reaktionstemperatur gehalten.

Dann wird ohne Abtrennung von Reaktionskomponenten das sekundäre Amin V zugegeben und die Umsetzung bei der Reaktionstemperatur und Reaktionszeit zuerst der Stufe b) und dann der Stufe c) durchgeführt. Nun führt man (d) das Lösungsmittel ab, fügt die wäßrige Säurelösung dem Destillationsrückstand zu und führt die Spaltung (e) bei der Reaktionstemperatur und Reaktionszeit der Stufe (e) durch. Der Endstoff kann aus dem Reaktionsgemisch dann in üblicher Weise, z.B. durch Abkühlen und Neutralisation des Gemischs, vorteilhaft auf einen pH von 7 bis 8, mit Basen, bevorzugt Ammoniak, und Filtration des gebildeten Niederschlags, isoliert werden.

Der Endstoff kann gegebenenfalls durch Wasserdampfdestillation oder/und Ausrühren mit Wasser, Essigester, Ethanol oder Methanol nachgereinigt werden.

Die nach dem Verfahren der Erfindung herstellbaren 1,1-disubstituierten Thioharnstoffe sind wertvolle Ausgangsstoffe für die Herstellung von Farbstoffen Pharmazeutika und Schädling sbekämpfungsmitteln. Bezüglich der Verwendung wird auf vorgenannte Veröffentlichung verwiesen.

**Beispiel 1**

$$(C_2H_5)_2N-\overset{S}{\overset{\|}{C}}-NH_2$$

In 450 Gramm Aceton wurden 81 Gramm Natriumisothiocyanat eingetragen. Danach wurden bei 5°C in 1 Stunde 108 Gramm Isobutylsäurechlorid zugegeben, 4 Stunden bei 5°C nachgerührt und 103 Gramm Diethylamin bei 12°C innerhalb 1 Stunde zugetropft. Nach Zugabe des Diethylamins wurde das Gemisch 4 Stunden bei 22°C nachgerührt. Dann wurde die gesamte Lösungsmittelmenge während 20 Minuten abdestilliert. Man gab 240 Gramm einer wäßrigen; 36 gew.%igen Salzsäure zu dem Rückstand. Das Gemisch wurde nun während 20 Minuten von 20 auf 90°C erhitzt und 60 Minuten auf 90°C gehalten. Das Gemisch wurde nun auf 25°C abgekühlt und mit 25 gew.%igen Ammoniak auf pH 7,2 gestellt. Der ausgefallene Endstoff wurde bei 5°C abfiltriert und mit 100 ml Eiswasser gewaschen. Man erhielt 107,4 Gramm (81,4 % der Theorie) N,N-Diethylthioharnstoff vom Pp. 102°C.

**Beispiel 2**

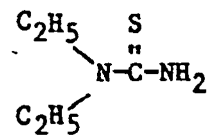

8,1 Gramm Natriumisothiocyanat wurden in 100 Gramm Aceton eingetragen. Zu dieser Suspension wurden bei 5°C in 1 Stunde 19,8 Gramm 1-Adanantancarbon-säurechlorid eingetragen. Das Gemisch wurde drei Stunden bei 5°C nachgerührt und danach 7,3 Gramm Diethylanin während 10 Minuten bei 12°C zugegeben. Nach Zugabe des Diethylamins wurde das Gemisch 4 Stunden bei 22°C nachgerührt. Dann wurde die gesamte Lösungsmittelmenge während 20 Minuten abdestilliert. Man gab 25 Gramm einer wäßrigen, 36 gew.%igen Salzsäure zu dem Rückstand. Das Gemisch wurde nun während 10 Minuten von 20 auf 95°C erhitzt und 60 Minuten auf 95°C gehalten. Das Gemisch wurde nun auf 25°C abgekühlt und mit 25 gew.%igem Ammoniak auf pH 7,5 gestellt. Der ausgefallene Endstoff wurde bei 5°C abfiltriert und zur Entfernung der Adamantansäure mit Essigester ausgerührt. Man erhielt 10,3 Gramm (78 % der Theorie) N,N-Diethylthioharnstoff vom Fp. 102°C.

**Beispiel 3**

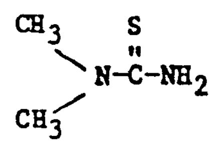

40,5 Gramm Natriumisothiocyanat wurden in 225 Gramm Aceton suspendiert. Zu der Suspension wurden bei 5°C während einer Stunde 60,4 Teile Pivalinsäurechlorid zugetropft und das Gemisch 3 Stunden bei 5°C nachgerührt. Danach wurden innerhalb von 50 Minuten 11,2 1 Dimethylamingas bei -8°C eingeleitet und das Gemisch 8 Stunden bei 22°C gerührt. Dann wurde die gesamte Lösungsmittelmenge während 20 Minuten abdestilliert. Man gab 130 Gramm einer wäßrigen, 36 gew.%igen Salzsäure zu dem Rückstand. Das Gemisch wurde nun binnen 20 Minuten von 25 bis 95°C erhitzt und während 60 Minuten bei 95°C gehalten. Das Gemisch wurde nun auf 25°C abgekühlt und mit 25 gew.%igem Ammoniak auf pH 7,5 gestellt. Der ausgefallene Endstoff wurde bei 5°C abfiltriert, mit wenig Eiswasser nachgewaschen und mit Essigester ausgerührt. Man erhielt 40 Gramm (77 % der Theorie) N,N-Dimethylthioharnstoff vom Fp. 166.

**Beispiel 4**

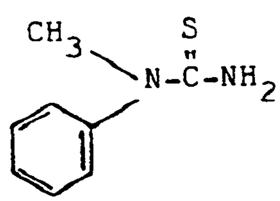

81 Gramm Natriumisothiocyanat wurden in 450 Gramm Aceton suspendiert. Zu der Suspension wurden bei 5°C 121 Gramm Pivalinsäurecblorid zugetropft und das Gemisch 3 Stunden bei 5°C nachgerührt. Danach wurden bei 12°C 107 Gramm N-Methylanilin während einer Stunde zugetropft; das Gemisch wurde 5 Stunden bei 22°C gerührt. Dann wurde die gesamte Lösungsmittelmenge während 30 Minuten abdestilliert. Man gab 240 Gramm einer wäßrigen, 36 gew.%igen Salzsäure zu dem Rückstand. Das Gemisch wurde nun binnen 20 Minuten von 25 bis 95°C erhitzt und während 90 Minuten bei 95°C gehalten. Das Gemisch wurde nun auf 25°C abgekühlt und mit 25 gew.%igem Ammoniak auf pH 7,5 gestellt. Der ausgefallene Endstoff wurde abfiltriert, mit Eiswasser nachgewaschen und mit Essigester ausgerührt. Man erhielt 12,4 Gramm (75 % der Theorie) N-Methyl-N-phenylthioharnstoff vom Fp. 115°C.

**Beispiel 5**

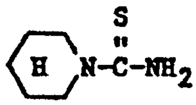

81 Gramm Natriumisothiocyanat wurden in 450 Gramm Aceton eingetragen. Zu dieser Suspension wurden bei 5°C in einer Stunde 121 Gramm Pivalinsäurechlorid zugetropft, das Gemisch 3 Stunden bei 5°C nachgerührt und danach bei 12°C 85 Gramm Piperidin während einer Stunde zugetropft. Nach Zugabe des Piperidins wurde das Gemisch während 4 Stunden bei 22°C nachgerührt. Dann wurde die gesamte Lösungsmittelmenge während 30 Minuten abdestilliert. Man gab 240 Gramm einer wäßrigen, 36 gew.%igen Salzsäure zu dem Rückstand. Das Gemisch wurde nun während 30 Minuten von 20 auf 95°C erhitzt und 90 Minuten auf 95°C gehalten. Das Gemisch wurde nun auf 25°C abgekühlt und mit 25 gew.%igem Ammoniak auf pH 7,5 gestellt. Der ausgefallene Endstoff wurde bei 5°C abfiltriert, mit Eiswasser nachgewaschen und mit Essigester ausgerührt. Man erhielt 112 Gramm (78 % der Theorie) Piperidinothioharnstoff vom Fp. 128°C.

**Beispiel 6**

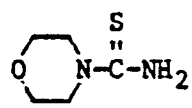

40,5 Gramm Natriumisothiocyanat wurden in 225 Gramm Aceton eingetragen. Zu dieser Suspension wurden bei 5°C in einer Stunde 60,4 Gramm Pivalinsäurechlorid zugetropft, das Gemisch 4 Stunden bei 5°C nachgerührt und danach bei 12°C 44 Gramm Morpholin während einer Stunde zugetropft. Nach Zugabe des Morpholins wurde das Gemisch während 4 Stunden bei 22°C nachgerührt. Dann wurde die gesamte Lösungsmittelmenge während 20 Minuten abdestilliert. Man gab 130 Gramm einer wäßrigen, 36 gew.%igen Salzsäure zu dem Rückstand. Das Gemisch wurde nun während 20 Minuten von 20 auf 95°C erhitzt und 75 Minuten auf 95°C gehalten. Das Gemisch wurde nun auf 25°C abgekühlt und mit 25 gew.%igem Anmoniak auf pH 7,5 gestellt. Der ausgefallene Endstoff wurde bei 5°C abfiltriert, mit Eiswasser nachgewaschen und mit Essigester ausgerührt. Man erhielt 59 Gramm (81 % der Theorie) Morpholinothioharnstoff vom Fp. 177°C.

**Beispiel 7**

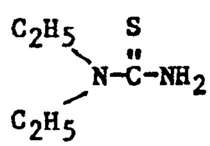

40,5 Gramm Natriumisothiocyanat wurden in 225 Gramm Aceton suspendiert. Zu der Suspension wurden bei 5°C 60,4 Gramm Pivalinsäurechlorid während einer Stunde zugetropft und das Gemisch 4 Stunden bei 5°C nachgerührt. Danach wurden innerhalb von 1 Stunde 36,3 Gramm Diethylamin bei 12°C zugetropft, das Gemisch wurde 4 Stunden bei 22°C gerührt. Dann wurde die gesamte Lösungsmittelmenge während 30 Minuten abdestilliert. Man gab 130 Gramm einer wäßrigen, 36 gew.%igen Salzsäure zu dem Rückstand. Das Gemisch wurde nun während 90 Minuten bei 90°C gehalten. Das Gemisch wurde nun auf 22°C abgekühlt und mit 25 gew.%igem Ammoniak auf pH 7,2 gestellt. Der ausgefallene Endstoff wurde bei 5°C abfiltriert, mit Eiswasser nachgewaschen und mit Essigester ausgerührt. Man erhielt 51 Gramm (77 % der Theorie) N,N-Diethylthioharnstoff vom Fp. 101°C.

**Beispiel 8**

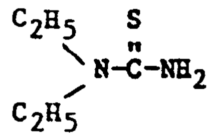

Man arbeitet analog Beispiel 7 und verwendet statt Aceton Essigester als Lösungsmittel. Ausbeute: 52,5 Gramm (79,5 % der Theorie) Diethylthioharnstoff vom Fp. 101° C.

**Beispiel 9**

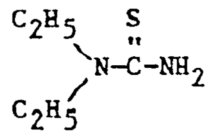

Man arbeitet analog Beispiel 7 und verwendet statt Aceton Toluol als Lösungsmittel. Ausbeute: 42,5 Gramm (64 % der Theorie) Diethylthioharnstoff, Fp. 101° C.

**Beispiel 10**

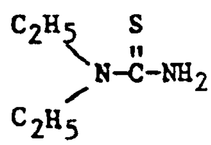

Man arbeitet analog Beispiel 7 und verwendet statt Aceton Methyl-tert.-butylether als Lösungsnittel. Ausbeute: 40 Gramm (61 % der Theorie) Diethylthioharnstoff, Fp. 101° C.

**Patentansprüche**

Verfahren zur Herstellung von 1,1-disubstituierten Thioharnstoffen der Formel

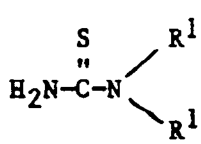

I,

worin die einzelnen Reste $R^1$ gleich oder verschieden sein können und jeweils einen aliphatischen, cycloaliphatischen, araliphatischen oder aromatischen Rest oder zusammen mit dem benachbarten Stickstoffatom Glieder eines heterocyclischen Ringes, der noch ein weiteres Stickstoffatom oder ein Sauerstoffatom enthalten kann, bedeuten, durch Umsetzung von Säurehalogeniden mit Isothiocyanaten in Gegenwart von organischen Lösungsmitteln, Umsetzung der gebildeten N-Acylisothiocyanate mit sekundären Aminen und Umsetzung der gebildeten 1,1-disubstituierten 3-Acylthioharnstoffe mit Säure und Isolierung des Endstoffes aus dem Gemisch in bekannter Weise, dadurch gekennzeichnet, daß man

a) in einer 1. Stufe Säurehalogenide der Formel

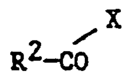 II,

worin $R^2$ einen verzweigten aliphatischen Rest, einen cycloaliphatischen Rest, einen an Benzolringen substituierten aromatischen Rest, einen araliphatischen Rest oder einen aromatisch-cycloaliphatischen Rest bedeutet, und X ein Halogenatom bezeichnet, mit Isothiocyanaten der Formel

$$R^3-S-C\equiv N \qquad III,$$

worin $R^3$ für ein Alkaliatom oder eine Ammoniumgruppe steht, in Gegenwart von organischen Lösungsmitteln bei einer Temperatur von höchstens 15°C umsetzt, dann

b) in einer 2. Stufe die so gebildeten N-Acylisothiocyanate der Formel

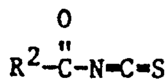 IV,

worin $R^2$ die vorgenannte Bedeutung besitzt, mit sekundären Aminen der Formel

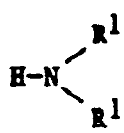 V,

worin $R^1$ die vorgenannte Bedeutung besitzt, bei einer Temperatur von höchstens 15°C während höchstens 90 Minuten unsetzt, dann

c) in einer 3. Stufe das Reaktionsgemisch während mindestens 180 Minuten bei einer Temperatur von 20 bis 35°C hält, dann

d) in einer 4. Stufe organische Lösungsmittel aus dem Reaktionsgemisch durch Destillation abtrennt, dann

e) in einer 5. Stufe zu den so gebildeten 1,1-disubstituierten 3-Acyl-thioharnstoffen der Formel

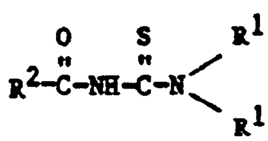 VI,

worin $R^1$ und $R^2$ die vorgenannte Bedeutung besitzen, 5 bis 38 gew.%ige, wäßrige Säuren in einem Verhältnis von höchstens 4 Äquivalenten Säure je Mol Stoff VI zugibt und das Gemisch bei einer Temperatur von 20 bis 100°C umsetzt.

## Claim

A process for the preparation of a 1,1-disubstituted thiourea of the formula

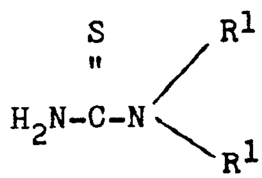 I,

where the individual radicals $R^1$ may be identical or different and are each an aliphatic, cycloaliphatic, araliphatic or aromatic radical, or, together with the adjacent nitrogen atom, are members of a heterocyclic ring which may contain a further nitrogen atom or an oxygen atom, by reacting an acid halide with an isothiocyanate in the presence of an organic solvent, reacting the resulting N-acylisothiocyanate with a secondary amine, and reacting the resulting 1,1-disubstituted 3-acylthiourea with an acid, and isolating the end product from the mixture in a conventional manner, wherein

(a) in a first stage, an acid halide of the formula

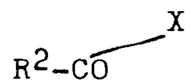

II,

where $R^2$ is a branched aliphatid radical, a cycloaliphatic radical, an aromatic radical substituted at benzene rings, an araliphatic radical or a cycloaliphatic radical bearing aromatic substituents, and X is halogen, is reacted with an isocyanate of the formula $R^3\text{-}S\text{-}C\equiv N$ III,

$$R^3-S-C\equiv N \qquad \text{III,}$$

where $R^3$ is an alkali atom or an ammonium group, in the presence of an organic solvent at a temperature of not more than 15°C,

(b) in a second stage, the resulting N-acylisothiocyanate of the formula

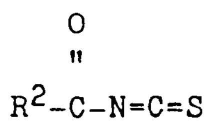

IV,

where $R^2$ has the above meanings, is reacted with a secondary amine of the formula

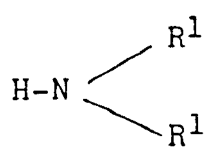

V,

where $R^1$ has the above meanings, at a temperature of not more than 15°C for not more than 90 minutes,

(c) in a third stage, the reaction mixture is kept at a temperature of from 20 to 35°C for at least 180 minutes,

(d) in a fourth stage, the organic solvent is separated from the reaction mixture by distillation, and

(e) in a fifth stage, there is added to the resulting 1,1-disubstituted 3-acylthiourea of the formula

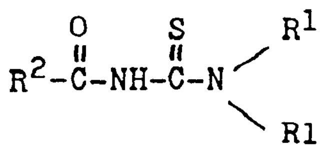

VI,

where $R^1$ and $R^2$ have the above meanings, a 5 to 38% strength by weight aqueous acid solution in a ratio of at most 4 equivalents of acid per mole of compound VI, and the mixture is reacted at a temperature of from 20 to 100°C.

**Revendication**

Procédé de préparation de thiourées 1,1-disubstituées répondant à la formule

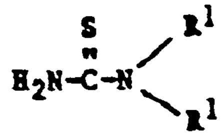 I,

dans laquelle les divers radicaux $R^1$ peuvent être identiques ou différents et désignent chacun un radical aliphatique, cycloaliphatique, araliphatique ou aromatique, ou forment avec l'atome d'azote voisin des maillons d'un cycle hétérocyclique, qui peut encore contenir un autre atome d'azote ou un atome d'oxygène, par réaction d'halogénures d'acide avec des isothiocyanates en présence de solvants organiques, réaction des N-acylisothiocyanates formés avec des amines secondaires et réaction des 3-acylthiourées 1,1-disubstituées formées avec un acide et isolement de la substance finale du mélange d'une manière connue, caractérisé en ce que

a) on fait réagir dans une première étape des halogénures d'acide répondant à la formule

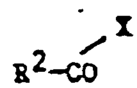 II,

dans laquelle $R^2$ désigne un radical aliphatique ramifié, un radical cycloaliphatique, un radical aromatique substitué sur les cycles benzéniques, un radical araliphatique ou un radical aromatique-cycloaliphatique, et X désigne un atome d'halogène, avec des isothiocyanates répondant à la formule

$$R^3-S-C\equiv N \qquad III$$

dans laquelle $R^3$ représente un atome de métal alcalin ou un groupe ammonium, en présence de solvants organiques à une température de 15° C au maximum, puis

b) on fait réagir dans une deuxième étape les N-acylisothiocyanates ainsi formés, répondant à la formule

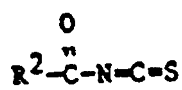 IV,

dans laquelle $R^2$ a la signification indiquée ci-dessus, avec des amines secondaires répondant à la formule

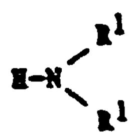 V,

dans laquelle $R^1$ a la signification indiquée ci-dessus, à une température de 15° C au maximum, pendant 90 minutes au maximum, puis

c) dans une troisième étape, on maintient le mélange réactionnel pendant 180 minutes au moins à une température de 20 à 35° C, puis

d) dans une quatrième étape, on sépare les solvants organiques du mélange réactionnel par distillation, puis

e) dans une cinquième étape, on ajoute aux 3-acylthiourées 1,1-disubstituées ainsi formées répondant à la formule

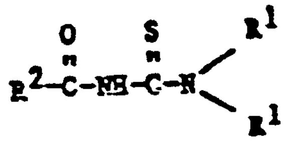 VI.

dans laquelle $R^1$ et $R^2$ ont la signification indiquée ci-dessus, 5 à 38 % en poids d'acides aqueux dans un rapport d'au plus 4 équivalents d'acide par mole de substance VI et on fait réagir le mélange à une température de 20 à 100° C.